# EUROPEAN PATENT SPECIFICATION

(11) **EP 4 188 902 B1**
(45) Date of publication and mention of the grant of the patent: **10.09.2025**
(21) Application number: 21758135.4
(22) Date of filing: 27.07.2021
(51) Int. Cl.: C07C 51/47, C07C 63/26, B01D 33/06

(54) **PRODUCTION OF PURIFIED TEREPHTHALIC ACID**
HERSTELLUNG GEREINIGTER TEREPHTHALSÄURE
PRODUCTION D'ACIDE TÉRÉPHTALIQUE PURIFIÉ

(30) Priority: 31.07.2020 US 202063059196 P
(43) Date of publication of application: 07.06.2023
(73) Proprietor: Koch Technology Solutions UK Limited, London EC2V 7AF (GB)
(72) Inventor: WARD, Philip N., Wichita, Kansas 67220 (US)
(74) Representative: Ter Meer Steinmeister & Partner
(86) International application number: PCT/IB2021/056796
(87) International publication number: WO 2022/023967

(56) References cited:
- WO-A1-2009/081458
- WO-A1-2015/077500
- WO-A1-2015/162281

## Description

### RELATED APPLICATIONS

The present application claims priority to U.S. Provisional Application Serial No. 63/059,196, filed July 31, 2020.

### FIELD

The present application relates to the production of purified terephthalic acid from crude terephthalic acid product obtained, for example, by paraxylene oxidation.

### BACKGROUND

WO 2015/077500 A1 relates to an integrated system for the preparation of terephthalic acid (PTA) and the use of the PTA for the production of poly(ethylene terephthalate) (PET). Methods of using the integrated system for the production are also described. The use of a rotary pressure filter (RPF) allows for the filtering of crude terephthalic acid (CTA) during solvent exchange and separately in filtering purified terephthalic acid (PTA) prior to product drying. Generally, a pneumatically (gas) driven RPF includes a filtration drum inside a pneumatically (gas) pressurized vessel. However, issues may arise with respect to gas pressure and circulation control within the RPF. Accordingly, there remains a significant interest in improving pneumatically driven RPF filtration in the production of PTA.

### SUMMARY

In one aspect, the present application provides a method for the production of purified terephthalic acid (PTA) as defined in claim 1. In embodiments, the method includes providing one or more pneumatically-driven rotary pressure filters, each having a rotating filter drum covered with a filtration media sitting partly submerged in a slurry trough; supplying a slurry comprising terephthalic acid to the trough of at least one pneumatically-driven rotary pressure filter, with the gas pressure difference across the rotating filter drum leading to a filter cake comprising terephthalic acid being formed on the rotating filter drum; and discharging the slurry into at least one collection vessel common to a plurality of RPFs, wherein a barometric conduit is connected to and forms a seal between the rotary pressure filter and the at least one collection vessel.

In another aspect, the present application provides a method as defined in claim 10. In embodiments, the method includes providing a plurality of pneumatically-driven rotary pressure filters, each having a rotating filter drum covered with a filtration media sitting partly submerged in a slurry trough; supplying a slurry comprising terephthalic acid to the trough of each of the pneumatically driven rotary pressure filters, with the gas pressure difference across the rotating filter drum leading to a filter cake comprising terephthalic acid being formed on the rotating filter drum; collecting overflow of the slurry in a first common vessel; discharging the filter cake into a second common vessel; and sealing each of the rotary pressure filters to the first common vessel and the second common vessel through barometric conduits.

In another aspect, the present application provides the use of an apparatus for controlling gas flow within and between a plurality of pneumatically-driven rotary pressure filters used in purified terephthalic acid production as defined in claim 12. In embodiments, the apparatus includes said filters are each connected to one or more common open-ended vessels, and wherein discharge from each of said plurality of rotary pressure filters is transferred to said one or more common vessels through at least one barometric conduit arranged between each of said filters and each of said one or more common open-ended vessels, wherein said at least one barometric conduit is configured to provide a liquid seal between each of said filters and each of said one or more common open-ended vessels.

### BRIEF DESCRIPTION OF THE DRAWINGS

FIG. 1 illustrates a pneumatically-driven rotary pressure filter (RPF) in accordance with aspects of the invention.
FIG. 2 illustrates a crude terephthalic acid (CTA) solvent interchange system in accordance with aspects of the invention.
FIGs. 3A-B illustrate a plurality of RPFs connected to at least one common vessel in accordance with aspects of the invention.
FIG. 4 illustrates a purified terephthalic acid (PTA) product recovery system in accordance with aspects of the invention.
FIG. 5 illustrates a process for obtaining PTA in accordance with aspects of the invention.

### DETAILED DESCRIPTION OF THE EMBODIMENTS

In the production of purified terephthalic acid (PTA), a pneumatically-driven rotary pressure filter (RPF) filters crude terephthalic acid (CTA) slurry during solvent interchange. For such pneumatically-driven RPFs, a rotating filtration drum covered with a filter medium sits partly submerged in a slurry trough within an open (not separated by any process valves or similar isolation devices) pressurized vessel, with a relatively low-pressure region within an inside region of the rotating filtration drum.

During the CTA solvent interchange process, the rotating filtration drum rotates in the slurry trough. As the filtration drum rotates, liquid from the slurry passes through the filter medium on the rotating filtration drum, thereby resulting in the formation of the CTA cake on the drum. Specifically, the CTA cake forms as gas pressure forces liquid through the CTA cake and filter medium from the outside region outside of the rotating filtration drum to the inside region within the rotating filtration drum. In embodiments, the outside region has a relatively higher pressure than the inside region. The CTA cake then undergoes washing and deliquoring as necessary as the filtration drum rotates. In embodiments, a slurry liquor within the slurry trough includes an acetic acid-based solvent.

Following the washing and deliquoring, the CTA cake discharges from the RPF through a discharge chute, prior to the rotating filtration drum passing back into the slurry trough. In embodiments, the discharge chute of the pneumatically-driven RPF links to an open re-slurry vessel operating in a pressure balance with the CTA cake discharge. In this way, the CTA cake drops into a common vessel for cake re-slurring in an aqueous solvent, prior to feeding the CTA cake into a CTA purification process. In further embodiments, the slurry trough includes an overflow which connects to common overflow vessels for receiving the overflow of slurry liquor from the slurry trough.

A solution for an increased PTA production demand includes implementing additional RPFs to increase a capacity for the filtering of the CTA to meet the demand. As an example, multiple parallel RPFs connect to a common re-slurry vessel. However, increasing the number of RPFs gives rise to various problems when the CTA cake discharges directly into the open re-slurry vessel and the overflow from the slurry trough connects to an open overflow vessel.

A first problem which arises is a need for the pneumatically-driven RPFs to operate at exactly the same pressure as each other in an attempt to avoid undesirable gas circulation patterns between the parallel pneumatically-driven RPFs. A second problem arises with respect to undesirable gas circulation patterns, which cause displacement of the acetic acid-based solvent of the slurry liquor within the RPF. This displacement of the acetic acid-based solvent results in the acetic acid vaporizing and contaminating clean wash water via vapor transport. Accordingly, gas circulation control is desirable for systems using a plurality of RPFs.

Attempts to control gas circulation patterns amongst the RPFs include feeding a relatively clean driving gas in the clean (washed) cake discharge area of each RPF for effecting filtration. However, additional gas circulation patterns between the pneumatically-driven RPFs and the open re-slurry vessels limit the effectiveness of this approach as gas supplies and pressures to the pneumatically-driven RPFs do not match.

The systems and processes described herein address the problems arising from using multiple RPFs by implementing barometric conduits between the RPFs and the re-slurry vessels and the overflow vessels. The barometric conduits provide liquid seals between the RPFs and common vessels, i.e., the open re-slurry vessels and the overflow vessels, by having the barometric conduits connect to the common vessels at a location below a liquid level within the common vessels. Accordingly, the liquid seals from the barometric conduits effectively control the gas circulation patterns and allow each individual RPF to operate at slightly different pressures (e.g., typically up to 0.25 bar difference) with respect to one another. In this way, the barometric conduits provide operational flexibility by allowing the different pressures. Specifically, the barometric conduits provide the benefit of making individual filter pressure control relatively less complex by not needing to be so precise in individual filter flow regulation.

In addition to providing gas flow control measures for the pneumatically-driven RPFs, the barometric conduits allow for the discharging of the CTA cake directly into the open re-slurry vessels. Further, the barometric conduits allow for the discharging of the slurry liquor from the slurry trough directly into the open re-slurry vessels. In embodiments, the individual barometric conduits also connect overflow lines from the slurry trough to common overflow vessels. In this way, in addition to gas circulation pattern control, the use of barometric conduits achieves pressure balancing as well by allowing the discharge of materials.

Described herein is a method for controlling gas flow within and between a plurality of pneumatically-driven rotary pressure filters used in purified terephthalic acid production, wherein said plurality of pneumatically-driven rotary pressure filters are each connected to one or more common vessels downstream of said plurality of pneumatically-driven rotary pressure filters, and said common vessels are open-ended. The method includes transferring discharge from each of said plurality of pneumatically-driven rotary pressure filters to said one or more common vessels directly downstream of said plurality of pneumatically-driven rotary pressure filters via at least one barometric conduit arranged between each of said plurality of pneumatically-driven rotary pressure filters and each of said one or more common vessels. The said at least one barometric conduit may be configured to provide a liquid seal between each of said plurality of pneumatically-driven rotary pressure filters and each of said one or more common vessels. In further embodiments, said plurality of pneumatically-driven rotary pressure filters are used for PTA filtration, and said one or more common vessels directly downstream of said filters is an overflow vessel. Said plurality of pneumatically-driven rotary pressure filters may be used for CTA filtration, and said one or more common vessels directly downstream of said plurality of pneumatically-driven rotary pressure filters includes an overflow vessel and a re-slurry vessel. In further embodiments, said one or more common vessels is integrally connected with each of said plurality of pneumatically-driven rotary pressure filters.

In embodiments of the use of an apparatus for controlling gas flow within and between a plurality of pneumatically-driven rotary pressure filters used in purified terephthalic acid production, said plurality of pneumatically-driven rotary pressure filters are each connected to one or more common vessels directly downstream of said plurality of pneumatically-driven rotary pressure filters. In further embodiments, said one or more common vessels are open-ended, and a discharge from each of said plurality of pneumatically-driven rotary pressure filters is transferred to said one or more common vessels directly downstream of said plurality of pneumatically-driven rotary pressure filters. The apparatus further includes at least one barometric conduit arranged between each of said plurality of pneumatically-driven rotary pressure filters and each of said one or more common vessels, wherein said at least one barometric conduit provides a liquid seal between each of said plurality of pneumatically-driven rotary pressure filters and each of said one or more common vessels. In embodiments, said plurality of pneumatically-driven rotary pressure filters is configured to be used for PTA filtration, and said one or more common vessels directly downstream of said plurality of pneumatically-driven rotary pressure filters is an overflow vessel. In further embodiments, said plurality of pneumatically-driven rotary pressure filters is configured to be used for CTA filtration, and said one or more common vessels directly downstream of said plurality of pneumatically-driven rotary pressure filters includes an overflow vessel and a re-slurry vessel. In embodiments, said one or more common vessels is integrally connected with each of said plurality of rotary pressure filters.

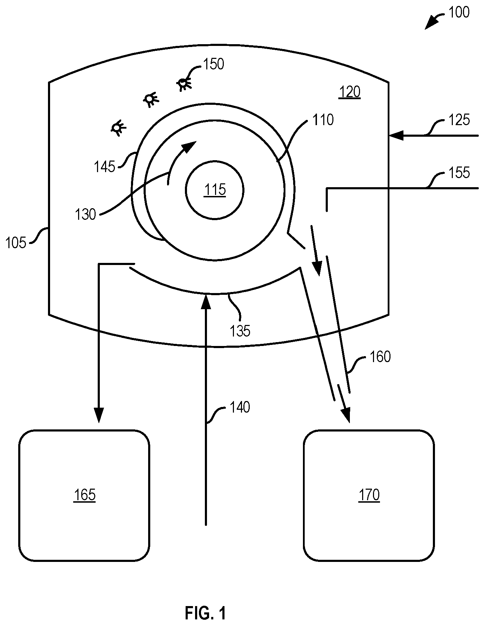

FIGS. 1-5 illustrate systems and processes for obtaining PTA. Specifically, FIG. 1 illustrates an RPF 100 in accordance with aspects of the present disclosure. In embodiments, the RPF 100 is a pneumatically (gas) driven pressure filter for filtering CTA and PTA materials. The RPF 100 includes a rotating filtration drum 110 within a pressurized vessel 105. The RPF 100 includes a relatively low-pressure region within an inside region 115 of the rotating filtration drum 110 and a relatively high-pressure region within an outside region 120 of the rotating filtration drum 110. "Relatively low-pressure region" and "relatively high-pressure region" indicate that the low-pressure region has a lower pressure than the relatively high-pressure region.

In operation, a driving gas 125 feeds into the pressurized vessel 105 as the rotating filtration drum 110 rotates within the vessel (in a clockwise direction 130 in the embodiment shown in FIG. 1). As the rotating filtration drum 110 rotates, it passes through a slurry of CTA in a solvent contained within a slurry trough 135 below the rotating filtration drum 110. Examples of the slurry solvent include an acetic acid-based solvent, amongst other examples. In embodiments, the slurry is fed into the slurry trough 135 through a slurry feed 140. In further embodiments, the slurry trough 135 includes an overflow which connects to a downstream overflow vessel 165 for the collection of slurry overflowing from the slurry trough 135.

As the filter drum 110 rotates through the slurry trough 135, the filtration drum 110 collects a layer of the CTA slurry, which then forms a CTA filter cake 145 after the rotating filtration drum 110 leaves the slurry trough 135. Specifically, the driving gas 125 deliquors the CTA by pushing liquid through the CTA from an outside region 120 outside of the rotating filtration drum 110 to an inside region 115 within the rotating filtration drum 110. In embodiments, the outside region 120 has a relatively higher pressure than the inside region 115.

In embodiments, following the CTA cake 145 formation, the CTA cake 145 undergoes further washing and deliquoring of the cake as necessary. In embodiments, washing jets 150 spray wash water onto the CTA cake 145 as the rotating filtration drum 110 rotates towards a discharge chute 160.

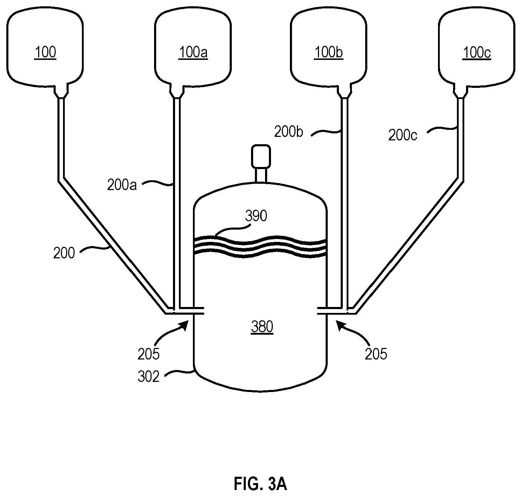

Continuing with FIG. 1, following the washing and deliquoring processes, the CTA cake 145 is discharged from the RPF 100 through a discharge chute 160, prior to the rotating filtration drum 110 passing back through the slurry trough 135. In embodiments, discharging of the CTA cake 145 includes applying clean water 155 to soften the CTA cake 145 and using a knife or scraper to remove the CTA cake 145 from the rotating filtration drum 110. In further embodiments, the discharge chute 160 links to an open re-slurry vessel 170 which is downstream from the RPF 100 and operates in a pressure balance with respect to the RPF 100. In this way, the CTA cake 145 drops into a common vessel for cake re-slurrying, prior to feeding the CTA cake 145 into a CTA purification process. Thus, overflow vessel 165 and re-slurry vessel 170 may each be referred to as a "collection vessel," where the overflow vessel 165 collects overflow slurry, and the re-slurry vessel 170 collects the CTA cake 145 (and, in some embodiments where the overflow vessel 165 is a multi-purpose "common" vessel downstream from multiple RPFs 100 as shown in Fig. 3A below, overflow slurry).

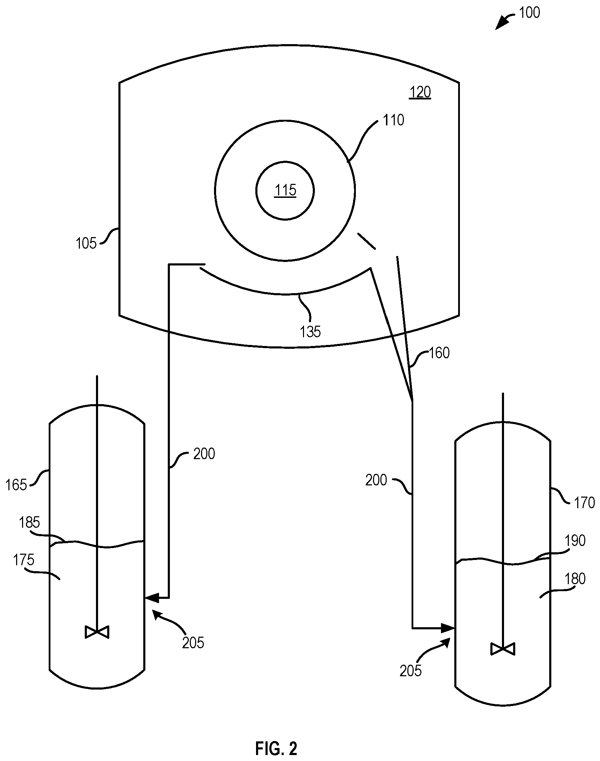

FIG. 2 illustrates barometric conduits 200 in accordance with aspects of the invention. As shown in FIG. 2, the barometric conduits 200 connect the RPF 100 to the overflow vessel 165 and the re-slurry vessel 170. In embodiments, the common vessels 165, 170 are open-ended vessels since they lack a metering valve. More specifically, the discharges from the overflow and the CTA cake 145 drop directly into the common vessels 165, 170, thereby making the common vessels 165, 170 open-ended.

The barometric conduits 200 connect to the common vessels 165, 170 below the liquid levels 185, 190 of the liquids 175, 180 within the vessels 165, 170. In embodiments, the liquid 175 is the overflow from the slurry trough 135, while the liquid 180 includes the solvent, such as water, for the re-slurrying of the discharged CTA cake 145. By connecting below the liquid levels 185, 190, the barometric conduits 200 form a liquid seal 205 between the RPF 100 and one or more vessels 165, 170. In this way, the one or more vessels 165, 170 are integrally connected with the RPF 100.

In embodiments, forming the liquid seal 205 allows for the RPF 100 to be sealed off from additional RPFs in filtering CTA. Accordingly, the RPF 100 is able to operate at a specific pressure, without being affected by gas flows from additional RPFs. In this way, the systems and processes described herein address the problems of controlling gas circulation patterns from a plurality of RPFs 100 by implementing barometric conduits 200. In further embodiments, the barometric conduits 200 allow for a control of a gas circulation pattern within a single RPF 100.

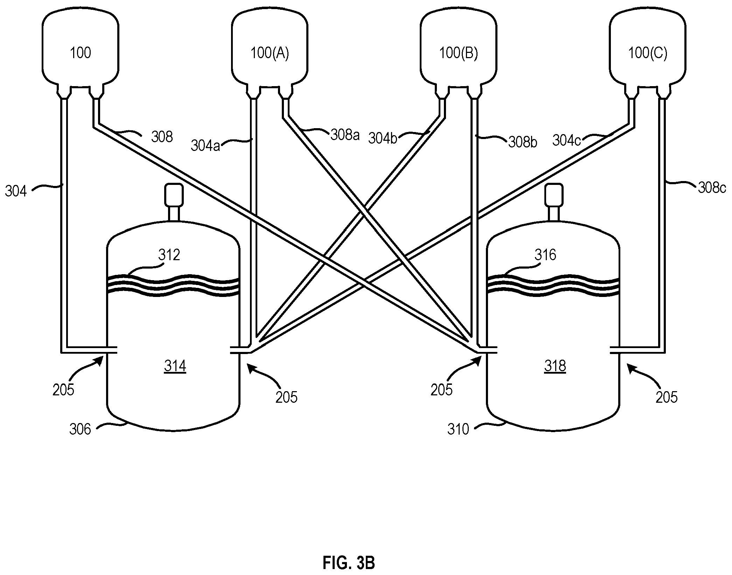

FIG. 3 illustrates a plurality of RPFs 100, 100a, 100b, 100c connecting to a multi-purpose common vessel 302 through barometric conduits 200 in accordance with aspects of the invention. As shown in FIG. 3, each RPF of the RPFs 100, 100a, 100b, 100c a respective barometric conduit 200, 200a, 200b, 200c connect each respective RPF 100, 100a, 100b, 100c. In this way, one or more common vessels 302 (which are, e.g., re-slurry vessel 170 or overflow vessel 165), are integrally connected with each of the plurality of RPFs 100, 100a, 100b, 100c.

The barometric conduits 200, 200a, 200b, 200c provide liquid seals 205 between the RPFs 100, 100a, 100b, 100c and the common vessels 302 (or, by having the barometric conduits 200, 200a, 200b, 200c connect to the common vessel 302 at a location below the liquid level 390 of the liquid 380 within the multi-purpose common vessel 302. Accordingly, the liquid seals 205 from the barometric conduits 200, 200a, 200b, 200c effectively control the gas circulation patterns coming from the RPFs 100, 100a, 100b, 100c, thereby allowing each individual RPF of the RPFs 100, 100a, 100b, 100c to operate at slightly different pressures. Although multiple of the barometric conduits 200 are shown coupled at the same junction into the common vessel 302 (e.g., in FIG. 3A, conduits 200, 200a into the left-side junction and conduits 200b, 200c into the right-side junction), it should be appreciated that each conduit may couple into the common vessel 302 at different locations, or all conduits may couple into the common vessel 302 at the same location.

In embodiments, the pressure difference between the RPFs 100, 100a, 100b, 100c can be, for example, up to 0.25 bar difference without uncontrolled and unwanted gas circulation patterns being established between the RPFs. In this way, the barometric conduits 200 provide operational flexibility by allowing different pressures throughout the RPFs 100, 100a, 100b, 100c. Accordingly, the barometric conduits 200, 200a, 200b, 200c provide the benefit of making individual filter pressure control relatively less complex by not needing to be so precise in individual filter flow regulation.

In embodiments, in addition to providing gas flow control measures for the RPFs 100, 100a, 100b, 100c, the barometric conduits 200, 200a, 200b, 200c allow for the discharging of the CTA cake 145 directly into the same common vessel 302.

In further embodiments, such as shown in FIG. 3B, the barometric conduits include an overflow barometric conduit 304, 304a, 304b, 304c that each allow for the discharging of the slurry liquor from the slurry trough directly into an overflow common vessel 306 (which is similar to overflow vessel 165 discussed above, but coupled to multiple RPFs), and a re-slurry barometric conduit 308, 308a, 038b, 308c that each allow for discharging of a CTA cake (e.g., CTA cake 145 discussed above) into a re-slurry vessel 310 (which is similar to re-slurry vessel 170 discussed above, but coupled to the multiple RPFs). In this way, in addition to gas circulation pattern control, the use of barometric conduits 200 achieves pressure balancing as well by allowing the discharge of materials into individual common vessels. Similar to the above discussion, each of overflow barometric conduit 304, 304a, 304b, 304c form a liquid seal 205 with the overflow common vessel 306 by being at a lower level than liquid level 312 of the liquid 314 within the overflow common vessel 306. Similar to the above discussion, each of re-slurry barometric conduit 308, 308a, 308b, 308c form a liquid seal 205 with the re-slurry common vessel 310 by being at a lower level than liquid level 316 of the liquid 318 within the re-slurry common vessel 310.

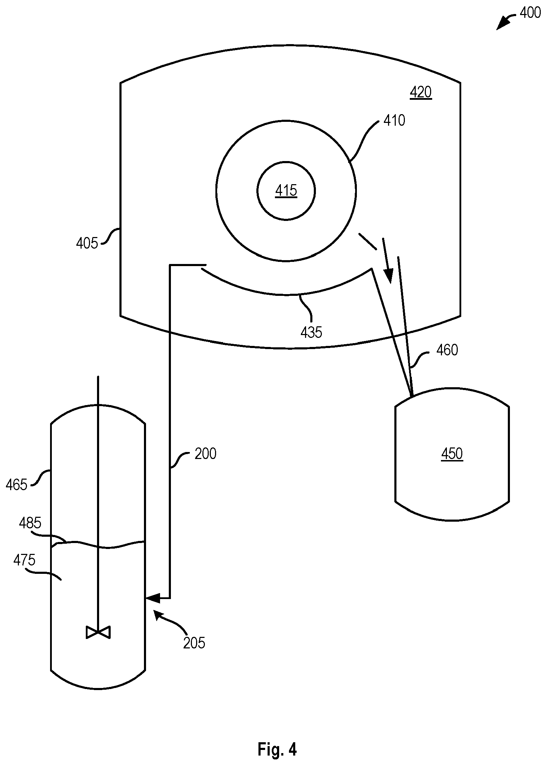

FIG. 4 illustrates a filtering of PTA slurry in accordance with aspects of the invention. In embodiments, the filtering of the PTA slurry includes implementation of barometric conduits 200 connecting an RPF 400 to the overflow vessel 465 below liquid level 485 of liquid 475 therein to create liquid seal 205. RPF 400 may be any of the above-discussed RPFs, including RPF 100, 100a, 100b, 100c. Overflow vessel 465 may be any of the above-discussed overflow vessel (e.g., overflow vessel 165), or common vessel (e.g., common vessel 302 or overflow common vessel 306 discussed above). Specifically, the RPF 400 includes a pressurized vessel 405 (which may be pressurized vessel 105, discussed above) and a rotating filtration drum 410 (which may be rotating filtration drum 110, discussed above) which rotates the PTA slurry for PTA cake formation. In embodiments, the rotating filtration drum 410 passes through the slurry trough 435 (which may be slurry trough 135, discussed above), forms a PTA cake on the drum due to the driving gas pressure difference between the outside and inside of the rotating filter drum, and gets washed and deliquored as described with respect to FIG. 1. In further embodiments, the slurry trough 435 includes an overflow which connects to a downstream overflow vessel 465 for the collection of slurry overflowing from the slurry trough 435 through the barometric conduit 200. Following the washing and deliquoring, the PTA cake is discharged through the discharge chute 460 (which may be discharge chute 160, discussed above) into a dryer 450 for obtaining PTA product. Dryer 450 may be moved to the outlet of discharge chute 460 (or 160) in place of re-slurry vessel 170, or any barometric conduit 200, 200a, 200b, 200c (or 308, 308a, 308b, 308c in FIG. 3B) to receive the PTA cake.

In the above description, each of vessels 165, 170, 302, 306, 310, and 465 may be referred to as a "collection vessel."

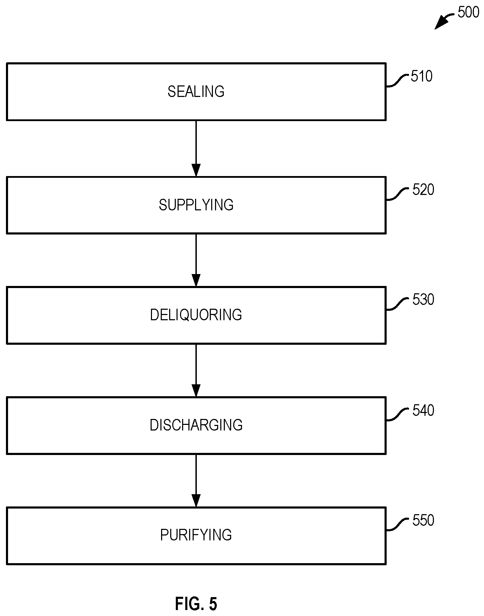

FIG. 5 illustrates an exemplary method 500 for filtering CTA in accordance with aspects of the invention. In step 510, there is a sealing of an RPF 100, 100a, 100b, and/or 100c with respect to common vessels 165, 170, 302, 306, and/or 310 downstream from the RPF 100, 100a, 100b, and/or 100c. In embodiments, and as described with respect to FIGS. 1-4, sealing 510 includes extending from barometric conduits 200 to a location at the common vessels 165, 170, 302, 306, and/or 310 below the liquid levels 185, 190, 390, 312, 316 of the liquids 175, 180, 380, 314, 318 within the common vessels 165, 170, 302, 306, 310, respectively. Thus, first barometric conduits 200 extend from the RPFs 100 to a common overflow vessel 165, and second barometric conduits 200 extend from the RPFs 100 to a common re-slurry vessel 170. In embodiments of step 510, the barometric conduits 200 are configured to allow for both the discharging of the slurry liquor from the slurry trough and CTA cake into the same multi-purpose common vessel (e.g., vessel 302 shown in Fig. 3A).

In step 520, there is a supplying of slurry within an RPF 100 (e.g., any of RPF 100, 100a, 100b, 100c discussed above). In embodiments, and as described with respect to FIGS. 1-4, a slurry trough 135 receives the slurry and the rotating filtration drum 110rotates through the slurry within the slurry trough 135.

In step 530, there is a deliquoring of the CTA slurry. In embodiments, and as described with respect to FIGS. 1-4, a driving gas 125 deliquors the CTA slurry as the drum rotates through the slurry trough 135, thereby forming the CTA cake 145. Specifically, the driving gas 125 leads to formation of the CTA cake by inducing slurry solvent to flow through the CTA cake and drum from an outside region 120 outside of the rotating filtration drum 110 to an inside region 115 within the rotating filtration drum 110. In embodiments, the outside region 120 has a relatively higher pressure than the inside region 115.

In step 540, there is a discharging of the CTA cake 145. In embodiments, and as described with respect to FIGS. 1-4, the CTA cake 145 discharges from the RPF 100, 100a, 100b, 100c through a discharge chute 160 and into an open re-slurry vessel 170, common vessel 302, or common re-slurry vessel 310 which is downstream from the RPF 100, 100a, 100b, and/or 100c. In embodiments, the discharge of the CTA cake 145 passes through the barometric conduit 200 (or, as shown in FIG. 3B, 308, 308a, 308b, 308c), which forms a liquid seal 205 with the re-slurry vessel 170, common vessel 302, or common re-slurry vessel 310 and allows for the control of gas circulation patterns within the RPF 100.

In step 550, there is a purifying of the PTA. In embodiments, and as described with respect to FIGS. 1-4, a PTA cake forms within the RPF 400, followed by discharging into a PTA Dryer 450 to obtain the PTA product. Step 550 may include moving the dryer 450 to the exit of discharge chute 160 discussed above. In further embodiments, the overflow from the slurry trough 135 (or 435 as in FIG. 4) discharges into an overflow vessel 165 (or a common vessel 302 as in FIG. 3A, or an overflow common vessel 306 as in FIG. 3B, or an overflow vessel 465 as in FIG. 4) through the barometric conduit 200 (or barometric conduits 304, 304a, 304b, 304c as in FIG. 3A).

While the present invention has been described and illustrated by reference to particular embodiments, those of ordinary skill in the art will appreciate that the invention lends itself to variations not necessarily illustrated herein. For this reason, then, reference should be made solely to the appended claims for purposes of determining the true scope of the present invention.

## Claims

1. A method for the production of purified terephthalic acid, the method comprising:
providing a pneumatically-driven rotary pressure filter having a rotating filter drum;
supplying a slurry comprising terephthalic acid to the rotary pressure filter such that a filter
cake comprising terephthalic acid is formed on the rotating filter drum; and discharging the slurry into an open-ended collection vessel via a barometric conduit that forms
a seal between the rotary pressure filter and the collection vessel,
wherein the seal between rotary pressure filter and the collection vessel is a liquid seal.

2. The method of claim 1, wherein the collection vessel is an overflow vessel.

3. The method of claim 1, wherein the barometric conduit connects to the collection vessel below a liquid level of a liquid within the collection vessel.

4. The method of claim 1, wherein the seal between the rotary pressure filter and the collection vessel is a liquid seal below a liquid level within the collection vessel.

5. The method of claim 1, further comprising the step of discharging said filter cake into a dryer for obtaining PTA product.

6. The method of claim 1, further comprising the step of discharging said filter cake into an open-ended re-slurry vessel for CTA solvent interchange.

7. The method of claim 6, wherein the seal between the rotary pressure filter and the re-slurry vessel is a liquid seal.

8. The method of claim 6, wherein the barometric conduit connects to the re-slurry vessel below a liquid level within the re-slurry vessel.

9. The method of claim 6, wherein the seal between the rotary pressure filter and the re-slurry vessel is a liquid seal below a liquid level within the re-slurry vessel.

10. A method for the filtering of CTA during solvent interchange in the production of purified terephthalic acid, the method comprising:
providing a plurality of rotary pressure filters, each having a rotating filter drum,
supplying a slurry comprising CTA to said plurality of rotary pressure filters such that a filter cake comprising CTA is formed on the rotating filter drum of each of the plurality of rotary pressure filters;
collecting overflow of the slurry in an open-ended first vessel common to each of the plurality of rotary pressure filters,
discharging the filter cake into an open-ended second vessel common to each of the plurality of rotary pressure filters;
sealing each of said rotary pressure filters to the first vessel and the second vessel through at least one barometric conduit, wherein sealing each of said rotary pressure filter to the first vessel and the second vessel includes forming a first liquid seal between each of said rotary pressure filters and a second liquid seal between each of said rotary pressure filters and the first vessel and the second vessel.

11. The method of claim 10, wherein:
each of said rotary pressure filters seals to the first vessel below a first liquid level within the first vessel, and
each of said rotary pressure filters seals to the second vessel below a second liquid level within the second vessel.

12. Use of an apparatus for controlling gas flow, within and between a plurality of pneumatically-driven rotary pressure filters used in terephthalic acid production, wherein said filters are each connected to one or more common vessels such that discharge from each of said plurality of rotary pressure filters is transferred to said one or more common vessels through at least one barometric conduit arranged between each of said rotary pressure filters and each of said one or more common vessels, wherein said at least one barometric conduit is configured to provide a liquid seal between each of said rotary pressure filters and each of said one or more common vessels.

13. Use of claim 12, wherein said one or more common vessels is integrally connected with each of said plurality of rotary pressure filters.

14. Use of claim 12 wherein said one or more common vessel includes an overflow vessel, and wherein said plurality of pneumatically-driven rotary pressure filters are preferably configured to be used for purified terephthalic acid (PTA) filtration.

15. Use of claim 12, wherein said one or more common vessels comprises an overflow vessel and a re-slurry vessel, and wherein said plurality of pneumatically-driven rotary pressure filters are preferably configured to be used for crude terephthalic acid (CTA) filtration.

16. Use of claim 12, wherein said one or more common vessels include a multi-purpose common vessel that is both an overflow vessel and a re-slurry vessel.

## Patentansprüche

1. Verfahren zur Herstellung von gereinigter Terephthalsäure, wobei das Verfahren
umfasst: Bereitstellen eines pneumatisch angetriebenen Druckdrehfilters, der eine rotierende Filtertrommel aufweist;
Zuführen einer Terephthalsäure umfassenden Aufschlämmung zu dem Druckdrehfilter, sodass auf der rotierenden Filtertrommel ein Terephthalsäure umfassender Filterkuchen gebildet wird; und
Ablassen der Aufschlämmung in einen Auffangbehälter mit offenem Ende über einen barometrischen Kanal, welcher eine Abdichtung zwischen dem Druckdrehfilter und dem Auffangbehälter bildet,
wobei die Abdichtung zwischen Druckdrehfilter und dem Auffangbehälter eine Flüssigkeitsabdichtung ist.

2. Verfahren nach Anspruch 1, wobei der Auffangbehälter ein Überlaufgefäß ist.

3. Verfahren nach Anspruch 1, wobei der barometrische Kanal unterhalb eines Flüssigkeitspegels einer Flüssigkeit innerhalb des Auffangbehälters mit dem Auffangbehälter verbunden ist.

4. Verfahren nach Anspruch 1, wobei die Abdichtung zwischen dem Druckdrehfilter und dem Auffangbehälter eine Flüssigkeitsabdichtung unterhalb eines Flüssigkeitspegels innerhalb des Auffangbehälters ist.

5. Verfahren nach Anspruch 1, weiter den Schritt des Ablassens des Filterkuchens in einen Trockner zum Gewinnen eines PTA-Produkts umfassend.

6. Verfahren nach Anspruch 1, weiter den Schritt des Ablassens des Filterkuchens in einen Wiederaufschlämmungsbehälter mit offenem Ende für CTA-Lösungsmittelaustausch umfassend.

7. Verfahren nach Anspruch 6, wobei die Abdichtung zwischen dem Druckdrehfilter und dem Wiederaufschlämmungsbehälter eine Flüssigkeitsabdichtung ist.

8. Verfahren nach Anspruch 6, wobei der barometrische Kanal unterhalb eines Flüssigkeitspegels innerhalb des Wiederaufschlämmungsbehälters mit dem Wiederaufschlämmungsbehälter verbunden ist.

9. Verfahren nach Anspruch 6, wobei die Abdichtung zwischen dem Druckdrehfilter und dem Wiederaufschlämmungsbehälter eine Flüssigkeitsabdichtung unterhalb eines Flüssigkeitspegels innerhalb des Wiederaufschlämmungsbehälter ist.

10. Verfahren zum Filtern von CTA während des Lösungsmittelaustauschs bei der Herstellung von gereinigter Terephthalsäure, wobei das Verfahren umfasst:
Bereitstellen einer Vielzahl von Druckdrehfiltern, die jeweils eine rotierende Filtertrommel aufweisen,
Zuführen einer CTA-umfassenden Aufschlämmung zu der Vielzahl von Druckdrehfiltern, sodass auf der rotierenden Filtertrommel jedes der Vielzahl von Druckdrehfiltern ein CTA-umfassender Filterkuchen gebildet wird;
Auffangen des Überlaufs der Aufschlämmung in einem ersten Behälter mit offenem Ende, welcher jedem der Vielzahl von Druckdrehfiltern gemeinsam ist,
Ablassen des Filterkuchens in einen zweiten Behälter mit offenem Ende, welcher jedem der Vielzahl von Druckdrehfiltern gemeinsam ist;
Abdichten jedes der Druckdrehfilter an dem ersten Behälter und dem zweiten Behälter durch zumindest einen barometrischen Kanal, wobei Abdichten jedes der Druckdrehfilter an dem ersten Behälter und dem zweiten Behälter Bilden einer ersten Flüssigkeitsabdichtung zwischen jedem der Druckdrehfilter und einer zweiten Flüssigkeitsabdichtung zwischen jedem der Druckdrehfilter und dem ersten Behälter und dem zweiten Behälter beinhaltet.

11. Verfahren nach Anspruch 10, wobei:
jeder der Druckdrehfilter den ersten Behälter unterhalb eines ersten Flüssigkeitspegels innerhalb des ersten Behälters abdichtet, und
jeder der Druckdrehfilter den zweiten Behälter unterhalb eines zweiten Flüssigkeitspegels innerhalb des zweiten Behälters abdichtet.

12. Verwendung einer Einrichtung zum Steuern des Gasflusses innerhalb und zwischen einer Vielzahl von pneumatisch angetriebenen Druckdrehfiltern, welche bei der Herstellung von Terephthalsäure verwendet werden, wobei die Filter jeweils mit einem oder mehreren gemeinsamen Behältern verbunden sind, sodass Ablassen von jedem der Vielzahl von Druckdrehfiltern durch zumindest einen barometrischen Kanal, welcher zwischen jedem der Druckdrehfilter und jedem des einen oder der mehreren gemeinsamen Behälter angeordnet ist, in den einen oder die mehreren gemeinsamen Behälter übertragen wird, wobei der zumindest eine barometrische Kanal dazu konfiguriert ist, eine Flüssigkeitsabdichtung zwischen jedem der Druckdrehfilter und jedem des einen oder der mehreren gemeinsamen Behälter bereitzustellen.

13. Verwendung nach Anspruch 12, wobei der eine oder die mehreren gemeinsamen Behälter einstückig mit jedem der Vielzahl von Druckdrehfiltern verbunden sind.

14. Verwendung nach Anspruch 12, wobei der eine oder die mehreren gemeinsamen Behälter ein Überlaufgefäß beinhalten, und wobei die Vielzahl von pneumatisch angetriebenen Druckdrehfiltern bevorzugt dazu konfiguriert sind, für die Filtration gereinigter Terephthalsäure (PTA) verwendet zu werden.

15. Verwendung nach Anspruch 12, wobei der eine oder die mehreren gemeinsamen Behälter ein Überlaufgefäß und einen Wiederaufschlämmungsbehälter umfassen, und wobei die Vielzahl von pneumatisch angetriebenen Druckdrehfiltern bevorzugt dazu konfiguriert sind, für die Filtration von roher Terephthalsäure (CTA) verwendet zu werden.

16. Verwendung nach Anspruch 12, wobei der eine oder die mehreren gemeinsamen Behälter einen gemeinsamen Mehrzweckbehälter beinhalten, welcher sowohl ein Überlaufgefäß als auch ein Wiederaufschlämmungsbehälter ist.

## Revendications

1. Procédé de production d'acide téréphtalique purifié, le procédé comprenant : la
fourniture d'un filtre rotatif sous pression à entraînement pneumatique comportant un tambour de filtre rotatif ;
la fourniture d'une suspension contenant de l'acide téréphtalique au filtre rotatif sous pression de telle sorte qu'un gâteau de filtration comprenant de l'acide téréphtalique soit formé sur le tambour de filtre rotatif ; et
la décharge de la suspension dans un récipient de collecte à extrémité ouverte au moyen d'un conduit barométrique qui forme un joint entre le filtre rotatif sous pression et le récipient de collecte,
dans lequel le joint entre le filtre rotatif sous pression et le récipient de collecte est un joint liquide.

2. Procédé selon la revendication 1, dans lequel le récipient de collecte est un récipient de trop-plein.

3. Procédé selon la revendication 1, dans lequel le conduit barométrique est relié au récipient de collecte en dessous d'un niveau de liquide d'un liquide à l'intérieur du récipient de collecte.

4. Procédé selon la revendication 1, dans lequel le joint entre le filtre rotatif sous pression et le récipient de collecte est un joint liquide en dessous d'un niveau de liquide à l'intérieur du récipient de collecte.

5. Procédé selon la revendication 1, comprenant en outre l'étape consistant à décharger ledit gâteau de filtration dans un séchoir pour obtenir un produit PTA.

6. Procédé selon la revendication 1, comprenant en outre l'étape consistant à décharger ledit gâteau de filtration dans un récipient de remise en suspension à extrémité ouverte pour un échange de solvant CTA.

7. Procédé selon la revendication 6, dans lequel le joint entre le filtre rotatif sous pression et le récipient de remise en suspension est un joint liquide.

8. Procédé selon la revendication 6, dans lequel le conduit barométrique est relié au récipient de remise en suspension en dessous d'un niveau de liquide à l'intérieur du récipient de remise en suspension.

9. Procédé selon la revendication 6, dans lequel le joint entre le filtre rotatif sous pression et le récipient de remise en suspension est un joint liquide situé en dessous d'un niveau de liquide à l'intérieur du récipient de remise en suspension.

10. Procédé de filtration de CTA lors d'un échange de solvant dans la production d'acide téréphtalique purifié, le procédé comprenant :
la fourniture d'une pluralité de filtres rotatifs sous pression, chacun présentant un tambour de filtre rotatif,
la fourniture d'une suspension comprenant du CTA à ladite pluralité de filtres rotatifs sous pression de telle sorte qu'un gâteau de filtration comprenant du CTA soit formé sur le tambour de filtre rotatif de chaque filtre rotatif sous pression de la pluralité de filtres rotatifs sous pression ;
la collecte du trop-plein de la suspension dans un premier récipient à extrémité ouverte commun à chacun des filtres rotatifs sous pression,
la décharge du gâteau de filtration dans un second récipient à extrémité ouverte commun à chaque filtre rotatif sous pression de la pluralité de filtres rotatifs sous pression ;
le scellage de chacun desdits filtres rotatifs sous pression au premier récipient et au second récipient au moyen d'au moins un conduit barométrique, dans lequel le scellage de chacun desdits filtres rotatifs sous pression au premier récipient et au second récipient inclut la formation d'un premier joint liquide entre chacun desdits filtres rotatifs sous pression et d'un second joint liquide entre chacun desdits filtres rotatifs sous pression et le premier récipient et le second récipient.

11. Procédé selon la revendication 10, dans lequel :
chacun desdits filtres rotatifs sous pression est scellé au premier récipient en dessous d'un premier niveau de liquide à l'intérieur du premier récipient, et
chacun desdits filtres rotatifs sous pression est scellé au second récipient en dessous d'un second niveau de liquide à l'intérieur du second récipient.

12. Utilisation d'un appareil pour réguler un flux de gaz, à l'intérieur et entre une pluralité de filtres rotatifs sous pression à entraînement pneumatique utilisés dans la production d'acide téréphtalique, dans laquelle lesdits filtres sont chacun reliés à un ou plusieurs récipients communs de telle sorte que la décharge de chaque filtre rotatif sous pression de ladite pluralité de filtres rotatifs sous pression soit transférée vers lesdits un ou plusieurs récipients communs au moyen d'au moins un conduit barométrique agencé entre chacun desdits filtres rotatifs sous pression et chacun desdits un ou plusieurs récipients communs, dans laquelle ledit au moins un conduit barométrique est configuré pour fournir un joint liquide entre chacun desdits filtres rotatifs sous pression et chacun desdits un ou plusieurs récipients communs.

13. Utilisation selon la revendication 12, dans laquelle lesdits un ou plusieurs récipients communs sont intégralement reliés à chaque filtre rotatif sous pression de ladite pluralité de filtres rotatifs sous pression.

14. Utilisation selon la revendication 12, dans laquelle lesdits un ou plusieurs récipients communs incluent un récipient de trop-plein et dans laquelle ladite pluralité de filtres rotatifs sous pression à entraînement pneumatique sont, de préférence, configurés pour être utilisés pour la filtration d'acide téréphtalique purifié (PTA).

15. Utilisation selon la revendication 12, dans laquelle lesdits un ou plusieurs récipients communs comprennent un récipient de trop-plein et un récipient de remise en suspension et dans laquelle ladite pluralité de filtres rotatifs sous pression à entraînement pneumatique sont, de préférence, configurés pour être utilisés pour la filtration d'acide téréphtalique brut (CTA).

16. Utilisation selon la revendication 12, dans laquelle lesdits un ou plusieurs récipients communs incluent un récipient commun polyvalent qui est à la fois un récipient de trop-plein et un récipient de remise en suspension.
